# EUROPEAN PATENT SPECIFICATION

(11) **EP 3 329 910 B1**
(45) Date of publication and mention of the grant of the patent: **14.12.2022**
(21) Application number: 16830659.5
(22) Date of filing: 16.02.2016
(51) Int. Cl.: A61K 9/00, A61K 9/06, A61K 47/32, A61K 9/70, A61P 31/10, A61K 31/045, A61K 31/17, A61K 31/194

(54) **PHARMACEUTICAL COMPOSITION FOR PREVENTING OR TREATING ONYCHOMYCOSIS AND PREPARATION METHOD THEREFOR**
PHARMAZEUTISCHE ZUSAMMENSETZUNG ZUR VORBEUGUNG ODER BEHANDLUNG VON NAGELMYKOSE UND HERSTELLUNGSVERFAHREN DAFÜR
COMPOSITION PHARMACEUTIQUE PERMETTANT DE PRÉVENIR OU DE TRAITER L'ONYCHOMYCOSE ET PROCÉDÉ DE PRÉPARATION ASSOCIÉ

(30) Priority: 30.07.2015 KR 20150107880
(43) Date of publication of application: 06.06.2018
(73) Proprietor: Wooshin Labottach Co., Ltd., Seoul 08390 (KR)
(72) Inventor: NAM, Tack-Soo, Seoul 07341 (KR)
(74) Representative: Turner, Craig Robert
(86) International application number: PCT/KR2016/001526
(87) International publication number: WO 2017/018625

(56) References cited:
- EP-A1- 2 777 689
- JP-A- H08 143 460
- KR-A- 20150 054 824
- KR-B1- 100 640 039
- KR-B1- 101 184 834
- US-A- 6 143 794
- L. EMTESTAM ET AL: "Treatment of distal subungual onychomycosis with a topical preparation of urea, propylene glycol and lactic acid: results of a 24-week, double-blind, placebo-controlled study : K101 treatment of onychomycosis", MYCOSES, vol. 55, no. 6, 11 June 2012 (2012-06-11), pages 532-540, XP055522015, GB ISSN: 0933-7407, DOI: 10.1111/j.1439-0507.2012.02215.x

## Description

### TECHNICAL FIELD

The present invention relates to a pharmaceutical composition for preventing or treating onychomycosis and a process for preparing the same. More specifically, the present invention relates to a pharmaceutical composition for preventing or treating onychomycosis, comprising urea; fumaric acid; and 1,3-butylene glycol as active ingredients, wherein the composition is in a hydrogel form and has keratolysis and moisture-retention abilities, as claimed in claim 1.

### BACKGROUND ART

Onychomycosis is caused by fungal infection of the nails and/or toenails, mainly by infection of *Trichophyton rubrum, Trichophyton mentagrophytes,* and the like. Systemic and topical treatments against onychomycosis are under way. Antifungal agents such as terbinafine and itraconazole have been used for the systemic treatment against onychomycosis, which may cause side effects including gastrointestinal side effects, headache, hepatotoxicity, and so on. Topical treatments include the use of lacquer formulations comprising antifungal agents such as amorolfine, ciclopiroxolamine, and the like. The locker formulation can be applied directly to the infected sites and thus has the advantage of avoiding systemic exposure to the drug.

As a composition for treating onychomycosis that does not contain an antifungal drug, there has been developed a topical formulation in the form of lacquer comprising urea, lactic acid, and propylene glycol (US 5,525,635). The topical formulation in the form of lacquer is currently commercially available as Emtrix^{™} (Ozhealth pharma). Urea is known to have a nail dissolving effect. Lactic acid is used as an additive in dermatological products where it is included mainly for its exfoliating properties. In addition, propylene glycol is known to have antibacterial and antimycotic properties as well as keratolytic effects. Although the mode of action of said topical formulation in the form of lacquer is not fully known, it is presumed to be mainly attributable to the osmotic effect of propylene glycol (L. Emtestam et al., Mycoses. 2012 Nov; 55(6): 532-540). And also, WO 2014/140524 has disclosed a formulation comprising a triol such as glycerol and a base for adjusting the pH to 2 to 6, in order to improve the storage stability of the topical formulation in the form of lacquer disclosed in US 5,525,635. EP 2777689 is derived from WO 2014/140524 and describes pharmaceutical compositions for topical application to the nail for treating nail diseases. The compositions comprise a urea-based component, a diol component, such as propylene glycol, an organic acid component, such as lactic acid, and a triol component, such as glycerol.

However, the known topical formulations for treating onychomycosis that does not contain an antifungal drug are in the form of lacquer, which makes it difficult to provide satisfactory keratolytic effects, and therefore have a limitation in exhibiting sufficient therapeutic activities.

### DISCLOSURE

### Technical Problem

The present inventor carried out various researches in order to develop an improved topical formulation for treating onychomycosis without containing antifungal drugs. Especially, the present inventor conducted various studies in order to develop a formulation capable of exhibiting sufficient keratolytic effects. As the results thereof, the present inventor has found that, when a high level of moisture content is maintained in the nail, drying phenomena in the nail is prevented and active ingredients are effectively penetrated into the skin, thereby being capable of increasing the keratolytic effects remarkably. Particularly, it has been found that, when urea in combination with a specific acid and diol compound, i.e., fumaric acid and 1,3-butylene glycol, is formulated to a hydrogel form containing at least about 45% of water, the resulting formulation exhibits remarkably excellent keratolytic activity as well as remarkably increased moisture-retention ability (moisture-maintenance ability).

Therefore, it is an object of the present invention to provide a pharmaceutical composition for preventing or treating onychomycosis, comprising urea, fumaric acid, and 1,3-butylene glycol, wherein the composition is in a hydrogel form and has keratolysis and moisture-retention abilities.

And also, it is another object of the present invention to provide a process for preparing the pharmaceutical composition in a hydrogel form..

### Technical Solution

In accordance with an aspect of the present invention, there is provided a pharmaceutical composition for preventing or treating onychomycosis, comprising urea; fumaric acid; 1,3-butylene glycol; a gel-forming polymer; a crosslinking agent; and 45 to 60 wt% of water, wherein the composition is in a hydrogel form and has keratolysis and moisture-retention abilities.The pharmaceutical composition of the present invention comprises 15 to 25 wt% of urea; 0.5 to 2.5 wt% of fumaric acid; 8 to 18 wt% of 1,3-butylene glycol; 5 to 10 wt% of the gel-forming polymer; 0.1 to 0.5 wt% of the crosslinking agent; and 45 to 60 wt% of water.

In accordance with another aspect of the present invention, there is provided a process for preparing a pharmaceutical composition having keratolysis and moisture-retention abilities for preventing or treating onychomycosis, the process of which comprises mixing 15 to 25 wt% of urea; 0.5 to 2.5 wt% of fumaric acid; 8 to 18 wt% of 1,3-butylene glycol; 5 to 10 wt% of a gel-forming polymer; 0.1 to 0.5 wt% of a crosslinking agent; 45 to 60 wt% of water; and optionally one or more excipients selected from the group consisting of a plasticizer, a thickening agent, a surfactant, a chelating agent and a preservative to form a hydrogel.

### ADVANTAGEOUS EFFECTS

It has been found by the present invention that, when a high level of moisture content is maintained in the nail, drying phenomena in the nail is prevented and active ingredients are effectively penetrated into the skin, thereby being capable of increasing the keratolytic effects remarkably. Particularly, it has been found by the present invention that, when urea in combination with a specific acid and diol compound, i.e., fumaric acid and 1,3-butylene glycol, is formulated to a hydrogel form containing at least about 45% of water, the resulting formulation exhibits remarkably excellent keratolytic activity as well as remarkably increased moisture-retention ability (moisture-maintenance ability). Therefore, the pharmaceutical composition according to the present invention having a hydrogel form has excellent keratolysis and moisture-retention abilities and therefore can be usefully applied for preventing or treating onychomycosis.

### DESCRIPTION OF DRAWINGS

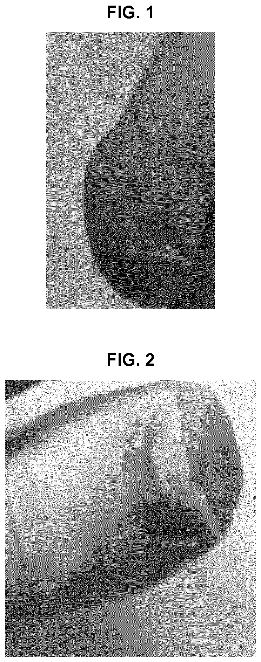

FIGs 1 to 5 are the results obtained by observing the toes after applying the patch obtained according to the present invention to a patient (42 years old, female) affected by onychomycosis. FIG. 1 shows the appearance of the toe before applying the patch and FIGs 2 to 5 show the appearances of the toe at 1 week, 2 weeks, 3 weeks, and 4 weeks after the attachment of the patch, respectively.

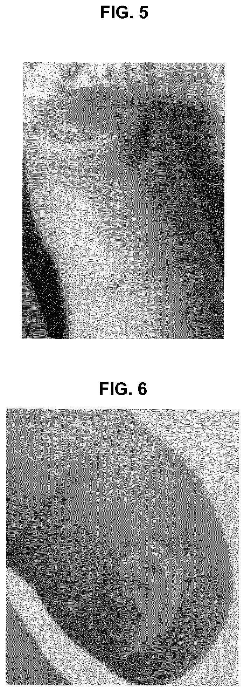

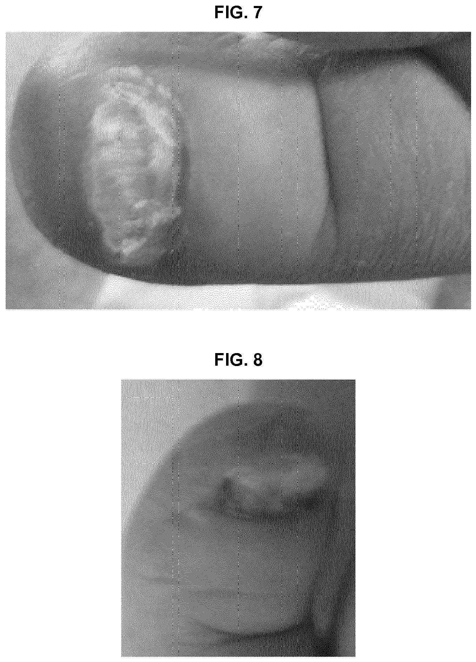

FIGs 6 to 10 are the results obtained by observing the toes after applying the patch obtained according to the present invention to a patient (35 years old, female) affected by onychomycosis. FIG. 6 shows the appearance of the toe before applying the patch and FIGs 7 to 10 show the appearances of the toe at 1 week, 2 weeks, 3 weeks, and 4 weeks after the attachment of the patch, respectively.

### BEST MODE

The present invention provides a pharmaceutical composition for preventing or treating onychomycosis, comprising urea; fumaric acid; 1,3-butylene glycol; a gel-forming polymer; a crosslinking agent; and 45 to 60 wt% of water, wherein the composition is in a hydrogel form and has keratolysis and moisture-retention abilities.

In the pharmaceutical composition of the present invention, the urea ispresent in an amount ranging from 15 to 25 wt%, preferably from 18 to 23 wt%, more preferably about 20 wt %, based on the total weight of the composition. And also, the fumaric acid is present in an amount ranging from 0.5 to 2.5 wt%, preferably from 1.0 to 2.3 wt%, more preferably about 2.0 wt %, based on the total weight of the composition. Said fumaric acid provides the viscosity suitable for forming a hydrogel, unlike other organic acids such as highly viscous and sticky lactic acid. And also, the 1,3-butylene glycol is present in an amount ranging from 8 to 18 wt%, preferably from 9 to 15 wt%, more preferably about 10 wt %, based on the total weight of the composition.

The pharmaceutical composition of the present invention comprises a gel-forming polymer and a crosslinking agent for crosslinking the gel-forming polymer. The gel-forming polymer may be polyacrylic acid, a copolymer of acrylic acid and sodium acrylate, or a mixture thereof. Said polyacrylic acid may have weight average molecular weight ranging from 100,000 to 400,000, preferably about 250,000. And also, said copolymer of acrylic acid and sodium acrylate may have weight average molecular weight ranging from 1,300,000 to 4,700,000, preferably about 3,000,000. More preferably, said copolymer may be a copolymer obtained by copolymerizing acrylic acid and sodium acrylate at a molar ratio of about 6 : 4. And also, a commercially available AP-40F^{™} (Sumintomo Seika Chemicals Co., Ltd.) may be used as a copolymer of acrylic acid and sodium acrylate. The gel-forming polymer ispresent in an amount sufficient for forming a hydrogel, in an amount ranging from 5 to 10 wt%, preferably 6 wt %, based on the total weight of the composition. The composition of the invention comprises 0.1 to 0.5 wt% of the crosslinking agent. As said crosslinking agent, aluminum glycinate may be preferably used. The aluminum glycinate may be present in amount ranging from 0.1 to 0.5 wt%, preferably about 0.2 wt%, based on the total weight of the composition.

The pharmaceutical composition of the present invention thus comprises 15 to 25 wt% of urea; 0.5 to 2.5 wt% of fumaric acid; 8 to 18 wt% of 1,3-butylene glycol; 5 to 10 wt% of the gel-forming polymer; 0.1 to 0.5 wt% of the crosslinking agent; and 45 to 60 wt% of water.

The pharmaceutical composition of the present invention may include a pharmaceutically acceptable excipient conventionally used in hydrogel formulations. For example, the pharmaceutical composition of the present invention may further comprise one or more excipients selected from the group consisting of a plasticizer, a thickening agent, a surfactant, a chelating agent and a preservative.

The plasticizer includes e.g., glycerin and may be present in an amount ranging from 3 to 8 wt%, based on the total weight of the composition. The thickening agent may be one or more selected from the group consisting of carboxymethyl cellulose or its salt, polyvinylpyrrolidone, and polyvinyl alcohol and may be present in an amount ranging from 5 to 10 wt%, based on the total weight of the composition. The surfactant may be one or more selected from the group consisting of sorbitan monooleate (for example, Span^{™} 80, etc.) and polyoxyethylene sorbitan fatty acid ester (for example, Tween^{™} 80, etc.) and may be present in an amount ranging from 0.3 to 1 wt%, based on the total weight of the composition. The chelating agent may be ethylenediaminetetraacetic acid or its salt and the preservative may be one or more selected from the group consisting of methylparaben and propylparaben. The amounts of said chelating agent and preservative are not specifically limited and may be appropriately selected by a person skilled in the art.

The present invention also provides a process for preparing the above pharmaceutical composition. That is, the present invention provides a process for preparing a pharmaceutical composition having keratolysis and moisture-retention abilities for preventing or treating onychomycosis, the process of which comprises mixing 15 to 25 wt% of urea; 0.5 to 2.5 wt% of fumaric acid; 8 to 18 wt% of 1,3-butylene glycol; 5 to 10 wt% of a gel-forming polymer; 0.1 to 0.5 wt% of a crosslinking agent; 45 to 60 wt% of water; and optionally one or more excipients selected from the group consisting of a plasticizer, a thickening agent, a surfactant, a chelating agent and a preservative to form a hydrogel.

In the process of the present invention, the urea, fumaric acid, 1,3-butylene glycol, gel-forming polymer, crosslinking agent, plasticizer, thickening agent, surfactant, chelating agent, and preservative are the same as described in the above.

The mixing may be performed by mixing and stirring all components at once under heating appropriately. And also, the mixing may be performed by preparing a plurality of mixed solutions containing some components and then mixing the mixed solutions. For example, the mixing may be carried out by preparing a solution containing polyvinylpyrrolidone and polyvinyl alcohol (Solution A); preparing a solution containing urea and fumaric acid (Solution B); preparing a mixed solution containing the remaining components; and mixing Solutions A and B to the mixed solution, followed by homogenizing the solution. The viscosity of the obtained hydrogel is preferably in the ranges of 100,000 to 2,000,000 cps at 25°C.

In the process of the present invention, the urea is used in an amount ranging from 15 to 25 wt%; the fumaric acid is used in an amount ranging from 0.5 to 2.5 wt%; the 1,3-butylene glycol is used in an amount ranging from 8 to 18 wt%; the gel-forming polymer is used in an amount ranging from 5 to 10 wt%; the crosslinking agent is used in an amount ranging from 0.1 to 0.5 wt%; and the water is used in an amount ranging from 45 to 60 wt%, based on the total weight of the composition.

The pharmaceutical composition of the present invention may be formulated to a dosage form such as patch. For example, the pharmaceutical composition of the present invention may be formulated to a patch form by casting the hydrogel obtained according to the present invention on a release layer and then forming a backing layer thereon. For the release layer, conventional release liners or their laminates may be used. For example, there may be used a film, a paper, or a laminates thereof, which made of polyethylene terephthalate, cast polypropylene, polyethylene, polyester, polyvinyl chloride, polyvinylidene chloride, etc. coated with silicone resin or fluorine resin. And also, conventionally used drug non-absorbable and flexible materials may be used as the backing layer (also referred to as "backing membrane"). For example, there may be used non-woven fabrics, non-woven fabrics coated with polyurethane, polyolefin, polyether, a multi-layer ethylene vinyl acetate film, polyester, polyurethane, etc.

In the pharmaceutical composition according to the present invention, for example in the pharmaceutical composition in a patch form, the active ingredients i.e., urea, fumaric acid, and 1,3-butylene glycol may be applied in the amounts of 75 to 225 mg, 7.5 to 22.5 mg, and 37.5 to 112.5 mg per topical application, respectively, but the amounts may vary depending on patient conditions, attached sites, and the like. And also, the pharmaceutical composition according to the present invention, for example the pharmaceutical composition in a patch form may be applied topically once a day, but not limited thereto. The application may continue for at least one week, at least two weeks, at least three weeks, at least four weeks, and at least six weeks, while exchanging for a new patch.

The present invention will be described in further detail with reference to the following examples and experimental examples. These examples and experimental examples are for illustrative purposes only and are not intended to limit the scope of the present invention.

### Formulation Examples: Preparation of hydrogels and patches comprising the same

Hydrogels were prepared according to the components and amounts shown in Table 1. The amounts of Table 1 represent the wt% of each component in the hydrogel. Polyvinylpyrrolidone and polyvinyl alcohol were added to water (about 60% of the total weight used), and the resulting mixture was stirred under heating to 50°C to obtain a solution (Solution A). Urea and fumaric acid (in case of Formulation Example 4, tartaric acid) were added to the remaining amount of water, and the resulting mixture was stirred under heating to 80°C to obtain a solution (Solution B). 1,3-Butylene glycol, polyacrylic acid (AC-10LHPK), the copolymer of acrylic acid and sodium acrylate (AP-40F), aluminum glycinate, glycerin, sodium carboxymethyl cellulose, Span^{™} 80, Tween^{™} 80, disodium ethylenediaminetetraacetate, methylparaben, and propylparaben were homogeneously mixed. Solutions A and B were added to the resulting solution, which was then homogeneously mixed for about 15 minutes. The resulting solution was casted on an embossed polypropylene film; and then a non-woven fabric coated with polyurethane for forming a backing membrane was laminated thereon to prepare obtain hydrogel-containing formulations having a patch form.

**<Table 1>**

| | Formulation Example 1 | Formulation Example 2 | Formulation Example 3 | Formulation Example 4 |
|---|---|---|---|---|
| Urea | 20.00 | 20.00 | - | 20.00 |
| Fumaric acid | 2.00 | 2.00 | 2.00 | - |
| Tartaric acid | - | - | - | 0.40 |
| 1,3-Butylene glycol | 10.00 | 10.00 | 10.00 | 10.00 |
| Water | 50.23 | 35.23 | 50.23 | 50.23 |
| Polyacrylic acid | 2.00 | 2.00 | 2.00 | 2.00 |
| Copolymer of acrylic acid and sodium acrylate | 4.00 | 4.00 | 4.00 | 4.00 |
| Aluminum glycinate | 0.20 | 0.20 | 0.20 | 0.20 |
| Glycerin | 5.00 | 20.00 | 25.00 | 6.60 |
| Sodium carboxymethyl cellulose | 2.50 | 2.50 | 2.50 | 2.50 |
| Polyvinylpyrrolidone | 0.80 | 0.80 | 0.80 | 0.80 |
| Polyvinyl alcohol | 2.50 | 2.50 | 2.50 | 2.50 |
| Span^{™} 80 | 0.25 | 0.25 | 0.25 | 0.25 |
| Tween^{™} 80 | 0.25 | 0.25 | 0.25 | 0.25 |
| Disodium ethylenediaminetetraacetate | 0.12 | 0.12 | 0.12 | 0.12 |
| methylparaben | 0.10 | 0.10 | 0.10 | 0.10 |
| propylparaben | 0.05 | 0.05 | 0.05 | 0.05 |
| Total | 100 | 100 | 100 | 100 |

### Formulation examples 2-4 are not according to the invention.

### Experimental Example 1: Evaluations on keratolysis and moisture-retention abilities

After the patches obtained in Formulation Examples 1 to 4 and the lacquer formulation (Emtrix^{™}, Ozhealth pharma) were respectively attached on the pig toenails for 5 hours, we evaluated the keratolysis and moisture-retention abilities. The evaluation on moisture-retention ability was performed by measuring the swellings due to the retention of moisture therein. Namely, the evaluation on moisture-retention ability was carried out by measuring the toenail thickness changes before and after the application of each formulation. The evaluation on keratolysis was performed by measuring the toenail hardness changes before and after the application of the formulation, with a texture analyzer. When the thickness and the hardness before application of each formulation are taken as 100%, the changes in the thickness and the hardness after application of each formulation are as shown in Tables 2 and 3 below.

**<Table 2>**

| | Formulation Example 1 | Formulation Example 2 | Formulation Example 3 | Formulation Example 4 | Emtrix^{™} |
|---|---|---|---|---|---|
| Thickness Change | 114.01% | 102.41% | 94.20% | 95.25% | 96.92% |
| Amount changed | 14.01% | 2.41% | -5.80% | -4.75% | -3.08% |

**<Table 3>**

| | Formulation Example 1 | Formulation Example 2 | Formulation Example 3 | Formulation Example 4 | Emtrix^{™} |
|---|---|---|---|---|---|
| Hardness Change | 78.96% | 99.70% | 86.93% | 84.01% | 99.28% |
| Amount changed | -21.04% | -0.30% | -13.07% | -15.99% | -0.72% |

As can be seen from the results of Table 2 above, the hydrogel formulation of Formulation Example 1 showed excellent moisture-retention ability. On the contrary, the formulations containing no urea or containing no fumaric acid (i.e., Formulation Examples 3 and 4) and the formulation in the form of lacquer (Emtrix^{™}) did not show moisture-retention ability. And also, the formulation containing about 35% of water (I.e., Formulation Example 2) did not exhibit significant moisture-retention ability.

And also, as can be seen from the results of Table 3 above, the hydrogel formulations exhibited relatively excellent keratolytic effects in comparison with the formulation in the form of lacquer (Emtrix^{™}) and the Formulation Example 1 containing fumaric acid exhibited the most excellent keratolytic effect.

### Experimental Example 2: Clinical Evaluations

The patch obtained in Formulation Example 1 was subjected to clinical tests in patients suffering from onychomycosis.

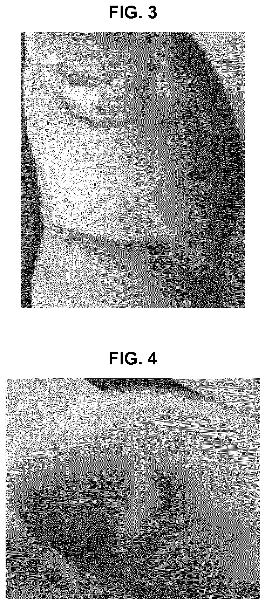

The patch of Formulation Example 1 was applied to a patient (42 years old, female) affected by onychomycosis, who was resistant to the itraconazole cream 1% (Sporanox). The patch was attached to the big toe of the left foot for 24 hours, and then a new patch was exchanged every day. The toes were photographed before the patch application (FIG. 1) and one week (FIG. 2), two weeks (FIG. 3), three weeks (FIG. 4) and four weeks (FIG. 5) after the patch application. The results thereof are shown in FIGs 1 to 5.

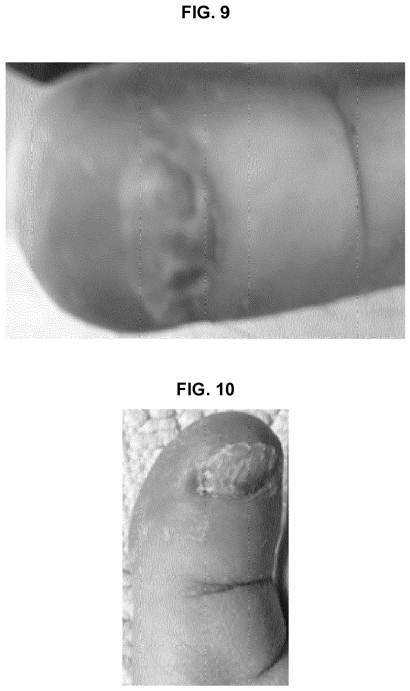

In addition, the patch of Formulation Example 1 was applied to a patient (35 years old, female) affected by onychomycosis, who was resistant to external and oral therapies. The patch was attached to the big toe of the left foot for 24 hours, and then a new patch was exchanged every day. The toes were photographed before the patch application (FIG. 6) and one week (FIG. 7), two weeks (FIG. 8), three weeks (FIG. 9) and four weeks (FIG. 10) after the patch application. The results thereof are shown in FIGs 6 to 10.

From the results of FIGs 1 to 10, it can be seen that the patch obtained according to the present invention has an excellent therapeutic activity against onychomycosis.

## Claims

1. A pharmaceutical composition for preventing or treating onychomycosis, comprising 15 to 25 wt% of urea; 0.5 to 2.5 wt% of fumaric acid; 8 to 18 wt% of 1,3-butylene glycol; 5 to 10 wt% of a gel-forming polymer; 0.1 to 0.5 wt% of a crosslinking agent; and 45 to 60 wt% of water, wherein the composition is in a hydrogel form and has keratolysis and moisture-retention abilities.

2. The pharmaceutical composition according to claim 1, wherein the gel-forming polymer is polyacrylic acid, a copolymer of acrylic acid and sodium acrylate, or a mixture thereof.

3. The pharmaceutical composition according to claim 1, wherein the crosslinking agent is aluminum glycinate.

4. The pharmaceutical composition according to any one of claims 1 to 3, further comprising one or more excipients selected from the group consisting of a plasticizer, a thickening agent, a surfactant, a chelating agent and a preservative.

5. The pharmaceutical composition according to claim 4, wherein the plasticizer is glycerin; and is present in an amount ranging from 3 to 8 wt%, based on the total weight of the composition.

6. The pharmaceutical composition according to claim 4, wherein the thickening agent is one or more selected from the group consisting of carboxymethyl cellulose or its salt, polyvinylpyrrolidone, and polyvinyl alcohol; and is present in an amount ranging from 5 to 10 wt%, based on the total weight of the composition.

7. The pharmaceutical composition according to claim 4, wherein the surfactant is one or more selected from the group consisting of sorbitan monooleate and polyoxyethylene sorbitan fatty acid ester; and is present in an amount ranging from 0.3 to 1 wt%, based on the total weight of the composition.

8. The pharmaceutical composition according to claim 4, wherein the chelating agent is ethylenediaminetetraacetic acid or its salt; and the preservative is one or more selected from the group consisting of methylparaben and propylparaben.

9. A process for preparing a pharmaceutical composition having keratolysis and moisture-retention abilities for preventing or treating onychomycosis, the process of which comprises mixing 15 to 25 wt% of urea; 0.5 to 2.5 wt% of fumaric acid; 8 to 18 wt% of 1,3-butylene glycol; 5 to 10 wt% of a gel-forming polymer; 0.1 to 0.5 wt% of a crosslinking agent; 45 to 60 wt% of water; and optionally one or more excipients selected from the group consisting of a plasticizer, a thickening agent, a surfactant, a chelating agent and a preservative to form a hydrogel.

## Patentansprüche

1. Pharmazeutische Zusammensetzung zur Vorbeugung oder Behandlung von Onychomykose, umfassend 15 bis 25 Gew.-% Harnstoff; 0,5 bis 2,5 Gew.-% Fumarsäure; 8 bis 18 Gew.-% 1,3-Butylenglycol; 5 bis 10 Gew.-% eines gelbildenden Polymers; 0,1 bis 0,5 Gew.-% eines Vernetzungsmittels; und 45 bis 60 Gew.-% Wasser, wobei die Zusammensetzung in Form von Hydrogel vorliegt und Keratolyse- und Feuchtigkeitsspeicherungsfähigkeiten aufweist.

2. Pharmazeutische Zusammensetzung nach Anspruch 1, wobei das gelbildende Polymer Polyacrylsäure, ein Copolymer aus Acrylsäure und Natriumacrylat oder eine Mischung davon ist.

3. Pharmazeutische Zusammensetzung nach Anspruch 1, wobei das Vernetzungsmittel Aluminiumglycinat ist.

4. Pharmazeutische Zusammensetzung nach einem der Ansprüche 1 bis 3, ferner umfassend einen oder mehrere Hilfsstoffe, ausgewählt aus der Gruppe bestehend aus einem Weichmacher, einem Verdickungsmittel, einem Tensid, einem Chelatbildner und einem Konservierungsmittel.

5. Pharmazeutische Zusammensetzung nach Anspruch 4, wobei der Weichmacher Glycerin ist und in einer Menge im Bereich von 3 bis 8 Gew.-%, bezogen auf das Gesamtgewicht der Zusammensetzung, vorliegt.

6. Pharmazeutische Zusammensetzung nach Anspruch 4, wobei das Verdickungsmittel eines oder mehrere ist, die ausgewählt sind aus der Gruppe bestehend aus Carboxymethylcellulose oder deren Salz, Polyvinylpyrrolidon und Polyvinylalkohol; und es in einer Menge im Bereich von 5 bis 10 Gew.-%, bezogen auf das Gesamtgewicht der Zusammensetzung, vorliegt.

7. Pharmazeutische Zusammensetzung nach Anspruch 4, wobei das Tensid eines oder mehrere ist, die ausgewählt sind aus der Gruppe bestehend aus Sorbitanmonooleat und PolyoxyethylensorbitanFettsäureester; und es in einer Menge im Bereich von 0,3 bis 1 Gew.-%, bezogen auf das Gesamtgewicht der Zusammensetzung, vorliegt.

8. Pharmazeutische Zusammensetzung nach Anspruch 4, wobei der Chelatbildner Ethylendiamintetraessigsäure oder deren Salz ist; und das Konservierungsmittel eines oder mehrere ist, die ausgewählt sind aus der Gruppe bestehend aus Methylparaben und Propylparaben.

9. Verfahren zum Herstellen einer pharmazeutischen Zusammensetzung, die Keratolyse- und Feuchtigkeitsspeicherungsfähigkeiten aufweist, zur Vorbeugung oder Behandlung von Onychomykose, wobei das Verfahren ein Mischen von 15 bis 25 Gew.-% Harnstoff; 0,5 bis 2,5 Gew.-% Fumarsäure; 8 bis 18 Gew.-% 1,3-Butylenglycol; 5 bis 10 Gew.-% eines gelbildenden Polymers; 0,1 bis 0,5 Gew.-% eines Vernetzungsmittels; 45 bis 60 Gew.-% Wasser; und optional eines oder mehrerer Hilfsstoffe, die ausgewählt sind aus der Gruppe bestehend aus einem Weichmacher, einem Verdickungsmittel, einem Tensid, einem Chelatbildner und einem Konservierungsmittel, zum Bilden eines Hydrogels umfasst.

## Revendications

1. Composition pharmaceutique destinée à la prévention ou au traitement de l'onychomycose, comprenant 15 à 25 % en poids d'urée ; 0,5 à 2,5 % en poids d'acide fumarique ; 8 à 18 % en poids de 1,3-butylèneglycol ; 5 à 10 % en poids d'un polymère gélifiant ; 0,1 à 0,5 % en poids d'un agent de réticulation ; et 45 à 60 % en poids d'eau, dans laquelle la composition se présente sous la forme d'un hydrogel et a des propriétés de kératolyse et de rétention d'eau.

2. Composition pharmaceutique selon la revendication 1, dans laquelle le polymère gélifiant est le polyacide acrylique, un copolymère d'acide acrylique et d'acrylate de sodium, ou un mélange de ceux-ci.

3. Composition pharmaceutique selon la revendication 1, dans laquelle l'agent de réticulation est le glycinate d'aluminium.

4. Composition pharmaceutique selon l'une quelconque des revendications 1 à 3, comprenant en outre un ou plusieurs excipients choisis dans le groupe constitué par un plastifiant, un agent épaississant, un tensioactif, un agent chélatant et un conservateur.

5. Composition pharmaceutique selon la revendication 4, dans laquelle le plastifiant est la glycérine ; et est présent en une quantité allant de 3 à 8 % en poids, par rapport au poids total de la composition.

6. Composition pharmaceutique selon la revendication 4, dans laquelle l'agent épaississant est un ou plusieurs agents choisis dans le groupe constitué par la carboxyméthylcellulose ou son sel, la polyvinylpyrrolidone et le polyalcool vinylique ; et est présent en une quantité allant de 5 à 10 % en poids, par rapport au poids total de la composition.

7. Composition pharmaceutique selon la revendication 4, dans laquelle le tensioactif est un ou plusieurs tensioactifs choisis dans le groupe constitué par le monooléate de sorbitane et un ester d'acide gras de polyoxyéthylène sorbitane ; et est présent en une quantité allant de 0,3 à 1 % en poids, par rapport au poids total de la composition.

8. Composition pharmaceutique selon la revendication 4, dans laquelle l'agent chélatant est l'acide éthylènediaminetétraacétique ou son sel ; et le conservateur est un ou plusieurs conservateurs choisis dans le groupe constitué par le méthylparabène et le propylparabène.

9. Procédé de préparation d'une composition pharmaceutique ayant des propriétés de kératolyse et de rétention d'eau destinée à la prévention ou au traitement de l'onychomycose, le procédé comprenant le mélange de 15 à 25 % en poids d'urée ; 0,5 % à 2,5 % en poids d'acide fumarique ; 8 à 18 % en poids de 1,3-butylène glycol ; 5 à 10 % en poids d'un polymère gélifiant ; 0,1 à 0,5 % en poids d'un agent de réticulation ; 45 à 60 % en poids d'eau ; et éventuellement un ou plusieurs excipients choisis dans le groupe constitué par un plastifiant, un agent épaississant, un tensioactif, un agent chélatant et un conservateur pour former un hydrogel.
